# EUROPÄISCHE PATENTSCHRIFT

(11) **EP 1 326 502 B1**
(45) Veröffentlichungstag und Bekanntmachung des Hinweises auf die Patenterteilung: **18.05.2005**
(21) Anmeldenummer: 01969667.3
(22) Anmeldetag: 29.08.2001
(51) Int. Cl.: A23L 1/302, A61K 31/205

(54) **VERFAHREN ZUR HERSTELLUNG EINER GRANULIERBAREN MISCHUNG UND CARNITIN-MAGNESIUM-HYDROXYCITRAT**
METHOD FOR PREPARING A MIXTURE THAT CAN BE GRANULATED AND CARNITINE-MAGNESIUM HYDROXYCITRATE
PROCEDE DE FABRICATION D'UN MELANGE POUVANT ETRE GRANULE ET HYDROXYCITRATE DE CARNITINE ET DE MAGNESIUM

(30) Priorität: 29.08.2000 EP 00118656
(43) Veröffentlichungstag der Anmeldung: 16.07.2003
(73) Patentinhaber: Lonza AG, 4052 Basel (CH)
(72) Erfinder: FUHRMANN, Martin, CH-4460 Gelterkinden (CH); PIANZOLA, Daniel, CH-3902 Brig-Glis (CH)
(86) Internationale Anmeldenummer: PCT/EP2001/009962
(87) Internationale Veröffentlichungsnummer: WO 2002/017735

(56) Entgegenhaltungen:
- EP-A- 0 402 755
- GB-A- 1 153 640
- US-A- 4 602 039
- US-A- 6 051 608

## Beschreibung

Die vorliegende Anmeldung betrifft ein neues Verfahren zur Herstellung einer granulierbaren Mischung, die für pharmazeutische Darreichungsformen oder zur Nahrungsergänzung geeignet ist, umfassend mindestens eine hygroskopische Substanz, vorzugsweise L-Carnitin. Weiterhin betrifft die Anmeldung solche neue granulierbare Mischungen umfassend Salze aus Carnitin, mindestens einem Alkali- oder Erdalkalimetallkation sowie einer organischen, physiologisch unbedenklichen Säure. Ein weiterer Gegenstand der vorliegenden Erfindung ist Carnitin-Magnesium-Hydroxycitrat.

L-(-)-Camitin kommt im eukaryontischen Organismus eine wichtige Rolle im Energiestoffwechsel, beim Fettsäureabbau, zu. Es handelt sich um eine vitaminähnliche Substanz, die seit längerem zur Vorbeugung und Behandlung von Mangelerscheinungen Lebensmitteln beigemischt wird oder als Nahrungsergänzung oder pharmazeutische Dareichungsform verabreicht wird. Insbesondere bei Kindern und älteren Menschen oder einseitiger Ernährung können derartige Mangelerscheinungen auftreten. Magnesium und Citrat sind ebenfalls physiologische Substanzen; insbesondere Mangelerscheinungen durch unzureichende Zufuhr von Magnesium sind nicht selten. Ihnen kann durch Aufnahme von Mg-Präparaten vorgebeugt werden. Citrat wird problemlos im Energiestoffwechsel verwertet. Das Muskelgewebe eines gesunden Erwachsenen enthält ungefähr 20 g Magnesium und 20 g L-Carnitin. Ein gesunder Erwachsener kann zur optimalen Energieversorung täglich rund 2 g L-Carnitin zusätzlich aufnehmen. Da Carnitin und Magnesium für eng miteinander zusammenhängende metabolische Prozesse benötigt werden, bewirkt ein Kombinationspräparat einen nützlichen synergistischen Effekt.

Es ist wohlbekannt, dass L-Carnitin eine hohe Hygroskopizität aufweist. Dies bedingt eine mangelnde Lagerfähigkeit der festen Substanz sowie daraus hergestellter, einfacher Pulvermischungen bzw. verursacht Probleme wie mangelnde Rieselfähigkeit bei der Weiterverarbeitung von reinem, festem Carnitin oder pulvrigen Mischungen mit Carnitin in der Nahrungsmittel-, Futtermittel- oder Pharmaindustrie.

EP-402 755 beschreibt das stöchiometrische Komplexsalz L-Carnitin-Magnesium-Citrat, das im Vergleich zum freien Carnitin eine deutlich geringere Hygroskopizität aufweist und somit lagerstabil ist. Bei 56% relativer Feuchtigkeit beträgt die Wasseraufnahme des Komplexsalzes nach 1 Woche Lagerung 21 Gew.%. Das Komplexsalz wird durch Vermischen der Komponenten in wässriger Lösung bei 60 °C hergestellt. Der Feststoff wird anschliessend durch Sprühtrocknung oder Kristallisation gewonnen.
Die Resthygroskopzität des so hergestellten Carnitin-Magnesium-Citrats erweist sich bei Lagerung und Weiterverarbeitung immer noch als ein gewisses Problem. Eine weitere Verringerung ist wünschenswert.

Es sind auch Salze bekannt, die neben Carnitin ausschliesslich Hydroxysäuren wie beispielsweise Weinsäure, ohne Beteiligung von Metallionen, umfassen, und die in ähnlicher Weise eine gegeüber dem reinen Carnitin verringerte Hygroskopzität aufweisen.
Alle bekannten Herstellungsverfahren bedingen sämtlich die Verwendung grösserer Mengen von Wasser oder wässrigen Lösungsmitteln und, zur Kristallisation, auch organischer Lösungsmittel. Die Trocknungsverfahren, z. B. Sprühtrocknung, erfordern einen erheblichen Energieaufwand oder die Verwendung organischer Lösungsmittel. Letzteres ist mit zusätzlichen Kosten und dem Problem der Entsorgung der Lösungsmittelabfälle behaftet.

Es ist die Aufgabe der vorliegenden Erfindung, diese und andere Nachteile des Standes der Technik zu vermeiden.

Diese Aufgabe wird durch das Herstellungsverfahren gemäss Anspruch 1 sowie durch Mischungen und Salze gemäss Ansprüchen 9 und 12 gelöst.

In einem erfindungsgemässen Verfahren zur Herstellung einer granulierbaren Mischung zur Nahrungsergänzung oder pharmazeutischen Verwendung, wobei die Mischung mindestens eine hygroskopische Substanz, vorzugsweise L-Carnitin, umfasst, wird in einer ersten Stufe die feste hygroskopische Substanz mit mindestens einer festen organischen Säure und mindestens einem festen Metallhydroxid ausgewählt aus der Gruppe umfassend Magesium- und Calcium hydroxid gemischt ohne Wasserzugabe oder unter Zugabe von höchstens 15 Gew.% Wasser, und in einer zweiten, anschliessenden Stufe der Wassergehalt der resultierenden Mischung durch Trocknung unter 5 Gew.% gesenkt, wobei die resultierende Masse vorzugsweise viskös bleibt und gegebenfalls in einem weiteren Verfahrensschritt verfestigt wird.
Unter Wasserzugabe im Sinne der vorliegenden Erfindung ist dabei das der Mischung gesamthaft in flüssiger Form zugeführte Wasser zu verstehen, ausschliesslich des Reaktionswassers der Neutralisationsreaktion zwischen Säure und Hydroxid. Es kann als separate Flüssigkeit zugefügt werden, es kann aber auch als Lösungs- oder Suspensionsmittel beispielsweise für die Säure oder das Hydroxid dienen; eine derartige Lösung oder Suspension kann dann dem Mischvorgang zugeführt werden. In diesem Sinne handelt es sich bei der organischen Säure und dem Metallhydroxid um an sich feste Ausgangsubstanzen, die der Mischung abgesehen vom eventuell vorhandenen Kristallwasser kein zusätzliches Wasser hinzufügen.

Unter festen Ausgangssubstanzen im Sinne der vorliegenden Erfindung sind vorzugsweise als grobzerteiltes Granulat oder feines Pulver vorliegende, rieselfähige Stoffe zu verstehen. Unter einer hygroskopischen Substanz in Sinne der vorliegenden Erfindung ist eine Substanz zu verstehen, die bei Lagerung unter Standardbedingungen durch Wasseraufnahme zum Verkleben oder Zerfliessen neigt. Insbesondere Substanzen, die nach Lagerung bei 56% relativer Feuchtigkeit nach 1 Woche unter Standardbedingungen über 20% Gewichtszunahme aufweisen, werden als hygroskopische Substanzen im Sinne der vorliegenden Anmeldung betrachtet. Derartige hygroskopische Substanzen sind z.B. Carnitin und Creatin, die stark Feuchtigkeit binden. Es kann sich aber auch um das Salz oder Derivat einer solchen hygroskopischen Verbindung handeln, beispielsweise um Carnitin-Chlorid oder ein Alkanoyl-Carnitin, insbesondere um Acetyl-Carnitin. Im Sinne der vorliegenden Erfindung handelt es sich bei Carnitin oder Alkanoyl-Carnitin bevorzugt um die physiologische L-(-)-Form. Es ist aber auch möglich, racemisches DL-Carnitin zu verwenden. Unter einer organischen Säure oder einem Säurerest im Sinne der vorliegenden Anmeldung sind insbesondere physiologisch unbedenkliche, ein- oder mehrwertige organische Säuren zu verstehen, insbesondere biogene Hydroxy- oder Fruchtsäuren und deren Derivate wie beispielsweise Ascorbinsäure (Vitamin C), Weinsäure, Äpfelsäure, Brenztraubensäure, Hydroxy-Brenztraubensäure, Fumarsäure, Glutarsäure, Zitronensäure, (-)-Hydroxy-Zitronensäure oder Isozitronensäure, Asparaginsäure, Glutaminsäure, Bernsteinsäure, wie sie z.B. in Brausetabletten und für den Gebrauch in Lebens- oder Arzneimitteln üblich und zugelassen sind. Unter einem Metallhydroxid im Sinne der vorliegenden Erfindung sind solche Hydroxide zu verstehen, die in fester Form vorliegen, nämlich die physiologisch unbedenkliche Erdalkalielemente Calcium oder Magnesium, die im Säugerorganismus in beträchtlicher Menge vorkommen.

Eine nach dem erfindungsgemässen Verfahren hergestellte, granulierbare Masse zeichnet sich überraschend durch eine besonders geringe Hygroskopizität im Vergleich zur Ausgangssubstanz, beispielsweise dem reinen Carnitin, aus. Ein weiterer Vorteil des erfindungsgemässen Verfahrens ist die Vermeidung der Zufuhr grosser Mengen Wasser, so dass zuletzt die Restfeuchtigkeit dem Endprodukt wieder aufwendig entzogen werden muss, sowie die einfache, keine zusätzlichen Aufarbeitungsschritte erfordernde Herstellungsweise.

Die Verwendung eines Metallhydroxids zusammen beispielsweise mit Zitronensäure oder einer anderen organischen Säure führt in einer Neutralisationsreaktion vorteilhaft und unmittelbar zu einer optimalen Verteilung des Reaktionswassers der Neutralisation zwischen den Feststoffpartikeln, und erlaubt so die Minimierung der Zugabe von Wasser sowie geeignete Fliesseigenschaften der pulvrigen bzw. pastösen Masse. In einer bevorzugten Ausführungsform beträgt der Mischungsanteil des zugefügten Wasser höchstens 3%, in einer besonders bevorzugten Ausführung höchstens 1 Gew. %, und in der am meisten bevorzugten Ausführungsform wird keine zusätzliches Wasser beigemischt. Lediglich *in situ* wird in geringer Menge Wasser, abhängig von den Mischungsverhältnissen, durch die Neutralisationsreaktion erzeugt. Erfindungsgemäss bewirkt dies eine schnellere Trocknung und geringere Hygroscopizität des festen Produktes.

Es ist auch möglich, der Mischung weitere, Substanzen wie Stabilisatoren, andere Wirkstoffe, pharmazeutische Trägerstoffe oder Füllstoffe, Wachse, Trockenmittel, Farbstoffe u.ä. beizumischen. Insbesondere ist das erfindungsgemässe Verfahren auch geeignet, Mischungen von in Bezug auf ihr Löslichkeitsverhalten wenig kompatiblen Feststoffen, z.B. hydrophoben und hydrophilen Substanzen wie beispielsweise Carnitin einerseits und fettlöslichen Vitaminen oder vitamin-artigen Substanzen wie Vitamin E oder Coenzym Q andererseits herzustellen.

Zum Vermischen der festen Ausgangssubstanzen können dem Fachmann an sich bekannte Mischvorrichtungen wie beispielsweise Knetmaschinen, wie sie für feste oder pastöse Substanzen üblicherweise verwandt werden, benutzt werden. Es ist auch möglich, beispielsweise geeignete Extruder wie z.B. einen Doppelschraubenextruder zu benutzen.Bevorzugt werden als Mischvorrichtung Knetmaschinen verwandt. Die Trocknung kann dann direkt im Gerät vorgenommen und bis zu einem gewünschten Vortrocknungsgrad, oder aber auch bis zur vollständigen Trocknung, vorgenommen werden.

Zweckmässigerweise weisen derartige Vorrichtungen geeignete Kontrollvorrichtungen zur Kontrolle der Produkttemperatur auf, da die anfängliche Neutralisationsreaktion zwischen Säure und Metallhydroxid zu starker Wärmeentwicklung führen kann. Die Produkttemperatur während des Mischvorganges der ersten Stufe sollte im wesentlichen, von einer kurzen Anfangsphase abgesehen, 50 °C-120 °C betragen. In einer bevorzugten Ausführungsform des Verfahrens sollte die Produkttemperatur in der ersten Stufe im wesentlichen 70°C -120°C betragen. Vorzugsweise wird zuerst die Säure- mit der Hydroxidkomponente vermischt, und nach der Neutralisationsreaktion die hygroskopische Substanz bzw. das Carnitin beigemischt. Die Produktemperatur sollte bei Zugabe der hygroskopische Substanz bzw. des Carnitins mindestens 50 °C, vorzugsweise mindestens 70 °C, im Rahmen der ersten Stufe des Verfahrens betragen. Die Reaktionsdauer beträgt vorzugsweise höchstens eine Stunde. Zweckmässigerweise werden die organische Säure und das Metallhydroxid in stöchiometrischen Mengen gemischt. Die Kombination von Wärme, feindispersen Feststoffen und einem geringen Anteil an Wasser als Lösungsmittel führt zu einer fliessfähigen, mischbaren Masse.

In einer bevorzugten Ausführungsform werden L-Carnitin, Magnesiumhydroxid sowie Zitronensäure in stöchiometrischen Mengen, d.h. in einem Molverhältnis von ungefähr 1:1:1, gemischt. Vorzugsweise handelt es sich um ausreichend reine, d.h. für die Lebensmittelproduktion oder die Herstellung von Pharmazeutika zugelassene Qualitäten dieser Substanzen, wie sie beispielsweise in der Europäischen Pharmacopöe beschrieben sind. Zweckmässigerweise werden wasserfreie Qualitäten verwandt. Zur Vermeidung einer unerwünschten Salzfracht wird das reine L-Carnitin der Mischung bevorzugt als neutrales, inneres Salz zugegeben.
Im Vergleich zum bisherigen Herstellungsverfahren ist die Hygroskopizität des so hergestellten Komplexsalzes L-Carnitin-Magnesium-Citrat weiter verringert. Beispielsweise beträgt die in dem Fachmann geläufiger Weise gravimetrisch bestimmte Feuchtigkeitsaufnahme von festem, zuvor im Vakuum an einer Ölpumpe oder über Phosphorpentoxid zur Gewichtskonstanz getrocknetem und nach dem erfindungsgemässen Verfahren hergestelltem Carnitin-Mg-Citrat nicht mehr als 7 Gew.% nach 48 h bei 56% relativer Feuchtigkeit (rH). Dieser Wert wird auch nach 330 h Lagerung nicht überschritten. Bezogen auf die in der Mischung enthaltene Menge an Carnitin oder Carnitinderivat, im Rahmen der Erfindung als Carnitingehalt, bezogen auf die freie Base, bezeichnet, entspricht das einer Feuchtigkeitsaufnahme von nicht mehr als 40 Gew.%. Vorzugsweise liegt in der erfindungsgemässen Mischung Carnitin-Mg-Citrat, bezogen auf den Mischungsanteil des Carnitins zu mindestens 80% als Komplexsalz vor.
Es ist auch möglich, in einer weiteren bevorzugten Ausführungsform ein Komplexsalz aus Carnitin, Magnesiumhydroxid und Hydroxy-Zitronensäure (HCA) herzustellen. Hydroxy-Zitronensäure ist weiter unten in der vorliegenden Beschreibung ausführlich beschrieben. Der HCA kommt eine spezielle Funktion als den Fettabbau förderndes Agens zu. Eine solche Kombination ist besonders vorteilhaft, weil die vorteilhaften stofflichen Eigenschaften des Komplexsalzes mit dem synergistischen Zusammenwirken seiner Komponenten einher geht.

Das erfindungsgemässe Verfahren erlaubt es auch, Mischungen von mit den bekannten Methoden nur schlecht kristallisierenden Salzen wie beispielsweise L-Carnitin und Ascorbinsäure herzustellen.

Vorzugsweise geschieht die Absenkung des Wassergehaltes der pastösen Mischung durch Vakuumtrocknung bei mindestens 85 °C, in einer am meisten bevorzugten Ausführungsform bei mindestens 90 °C, und höchstens 120 °C, und erfolgt bei einem Druck von bis zu 25 mbar, vorzugsweise nicht mehr als 50 mbar. Die Trocknung kann, in Abhängigkeit von der Produktmenge, ungefähr bis zu 3 h dauern. Überraschenderweise ist die so erzeugte Masse, trotz ihrer zunehmenden Verfestigung bei abnehmendem Wassergehalt, immer noch in sehr beschränktem Masse fliessfähig bzw. pastös. Als pastös im Sinne der vorliegenden Erfindung gilt ebenso eine nur noch unter erhöhtem Druck (>1 bar) und bei mindestens 85 °C fliessfähige Masse, im Sinne eines als Extrusionsviskosität bezeichneten Fliessverhaltens. Die pastöse Masse kann durch geeignete Vorrichtungen mit einem ausreichendem Drehmoment im heissen Zustand bewegt werden. Eine endgültige Verfestigung tritt erst durch Abkühlung ein. Es ist möglich, die Abkühlung durch an sich bekannte Verfahren, beispielsweise Extrusion als dünner Strang und/oder Erstarrung auf einem Kühlband, zu beschleunigen. Es ist auch möglich, einen zusätzlichen Nachtrocknungsschritt während oder nach dem Abkühlungsvorgang hinzuzufügen, beispielsweise eine weitere Vakuumtrocknung. Bei Verwendung beispielsweise von Knetmaschinen mit geringem Drehmoment bzw. einer für den Austrag ungünstigen konstruktiven Ausführung kann es zweckmässig sein, die noch heisse Masse bereits vor dem ersten Vakuumtrocknungsschritt im noch zähflüssigen Zustand aus der Mischvorrichtung auszutragen und dann erst die weiteren Bearbeitungschritte gemäss dem erfindungsgemässen Verfahren vorzunehmen.

Das einmal erstarrte Produkt kann zur Weiterverarbeitung grob zerkleinert und einer End- bzw. Feingranulation zugeführt werden. Es kann in einer bevorzugten Ausführung des vorliegenden Verfahrens zu einer Partikelgrösse von höchstens 1 nun mit dem Fachmann an sich bekannten Vorrichtungen zerkleinert werden. In einer weiteren bevorzugten Ausführungsform wird die Zerkleinerung mit Siebgranulatoren (Fa. FREWITT) vorgenommen, vorzugsweise bei Verwendung eines Extruders mit geeigneten Düsenplatten unmittelbar mit dem vom Extruder erzeugten Grobgranulat ohne zusätzlichen zwischengeschalteten Zerkleinerungsschritt.

Es ist auch möglich, im erfindungsgemässen Verfahren weitere Wirksubstanzen der erfindungsgemässen Mischung während des Mischvorganges hinzuzufügen, beispielsweise Coenzyme wie Niacin oder Niacinamid oder andere leistungssteigemde oder den Fettabbau fördernde Substanzen, die synergistisch mit L-Carnitin zusammenwirken können, wie β-Hydroxy-β-methyl-butyrate, (-)-Hydroxycitrat, Liponsäure oder lipotrophe Substanzen wie Lecithin oder Cholin. Ca²⁺ wie Mg²⁺ werden für die Muskelaktivität benötigt, so dass sich hier ein geeigneter synergistischer Effekt mit der die Muskeltätigkeit bzw. den Energiestoffwechsel fördernden Wirkung von Carnitin oder auch Creatin ergibt.

Ein weiterer Gegenstand der vorliegenden Anmeldung ist eine granulierbare Mischung, gemäss Anspruch 9, vorzugsweise zur Nahrungsergänzung oder zur pharmazeutischen Dosierung, umfassend Salze aus Carnitin, vorzugsweise L-Carnitin, mindestens einem Alkali- oder Erdalkalimetall sowie einer physiologisch unbedenklichen organischen Säure, dadurch gekennzeichnet, dass die Wasseraufnahme der zuvor getrockneten Mischung bei 56% relativer Feuchtigkeit bei Atmosphärendruck und 25°C nach 24 h, vorzugsweise auch noch nach 14 Tagen, höchstens 15 Gew.%, vorzugsweise höchstens 10 Gew.%, und in einer am meisten bevorzugten Ausführungsform höchstens 7 Gew.% beträgt. Bezogen auf den Carnitingehalt der Mischung beträgt die Wasseraufnahme der zuvor getrockneten Mischung höchstens 40 Gew.%. Unter Trocknung wird in diesem Zusammenhang die Trocknung über Phosphorpentoxid zur Gewichtskonstanz bei 25°C verstanden. Unter Salzen werden in diesem Zusammenhang alle möglichen Salzverbindungen zwischen jeweils mindestens zweien der in der erfindungsgemässen Mischung vorhandenen Substanzen verstanden. Eine solche Mischung ist nach dem erfindungsgemässen Verfahren herstellbar und weist sich durch eine besonders niedrige Hygroskopizität aus. Insbesondere bei Carnitin-Magnesium-Citrat oder -Hydroxycitrat ist diese gegenüber der mit dem bisherigen Herstellungsverfahren erzeugten Qualität weiter verringert.

Ensprechend ist in einer bevorzugten Ausführungsform die organische Säure bzw. der in der Mischung vorliegende Säurerest vorzugsweise ein Citrat, ein (-)-Hydroxycitrat oder ein Ascorbat und, vorzugsweise, das Erdalkalimetall Calcium, Magnesium oder eine Mischung beider. Darüberhinaus liegen der Säurerest sowie das Erdalkalimetallkation in einer bevorzugten Ausführungsform in stöchiometrisch äquivalenten Mengen vor. In einer weiteren bevorzugten Ausführungsform enthält die Mischung zusätzlich mindestens eine weitere Substanz aus der Gruppe bestehend aus Ribose, Niacin, Niacinamid, β-Hydroxy-β-methylbutyrat, Liponsäure, Coenzym Q 10 und Chrom(III) Salze, beispielsweise Chrom-Picolinate oder Chrom-Nicotinate, umfasst.

Ein weiterer Gegenstand der vorliegenden Erfindung ist eine Mischung zur Nahrungsergänzung umfassend L-Carnitin oder ein Alkanoyl-Carnitin, Coenzym Q und β-Hydroxy-β-methyl-butyrat sowie, optional, (-)-Hydroxycitrat. Es kann sich auch um eine Kombination verschiedener Carnitin-Salze einschliesslich der inneren Salze bzw. der Carnitin-Derivate sowie deren Salze handeln. Eine derartige Mischung ist neu und kann gleichfalls in einem erfindungsgemässen Feststoffmischverfahren, beispielsweise mit einem Extruder, als granulierbare Mischung hergestellt werden. Eine derartige Mischung kann aber auch in einem anderen bekanntem Verfahren hergestellt werden.

Die in dieser Mischung enthaltenen organischen Säuren können in der protonierten Form oder als Salz mit einem Gegenion in der Mischung vorliegen. Insbesondere das Carnitin oder Carnitin-Derivat kann als Komplexsalz oder als inneres Salz oder als Salz mit einem Gegenion in der Mischung vorliegen. Der Vorteil einer solchen Mischung liegt im synergistischen Zusammenwirken der vorteilhaften Eigenschaften bzw. physiologischen Funktionen der Komponenten.
Die erfindungsgemässe Mischung umfasst sowohl Stoffe, die die Fettverbrennung bzw. die Mobilisierung des Körperfetts fördern, wie auch die Neusynthese von Fett bzw. Fettsäuren hemmende Substanzen. Eine derartige Mischung kann vor allem als den Abbau von Körperfett unterstützende Nahrungsergänzung oder, im Rahmen einer Diät, als Bestandteil von Nahrungsersatzzubereitungen oder Schlankheitsmitteln dienen.

Carnitin, d.h. in seiner physiologisch wirksamen Form das L-(-)-Enantiomere, fördert den Transport von Fett bzw. von dem metabolischen Grundbaustein Acetyl-CoA, durch Zellmembranen hindurch zum Ort der Verwertung im Energiestoffwechsel, der Mitochondrienmatrix. Carnitin erhöht also den Stofffluss im Rahmen der beta-Oxidation der Fettsäuren. Ubichinon bzw. Coenzym Q, vorzugsweise Coenzym Q10, ist ein weiterer membrangängiges Molekül, das im Rahmen der Redoxvorgänge der oxidativen Phosphorylierung ein wichtiges Zwischenglied als Elektronenüberträger in der Mitochondrienmembran und damit in der Verwertung der durch die Fettsäureverbrennung erzeugten metabolischen Energie ist. Die Kombination von Carnitin und Ubichinon stellt somit ein vorteilhaftes, kumulatives Zusammenwirken zweier Wirkprinzipien dar.
Gleichzeitig wird die Neusynthese von Fettsäuren sowie die Verwertung alternativer Energiequellen im Rahmen des Citratcyclus, behindert. (-)-Hydroxycitrat hemmt die ATP-Citrat-Lyase. β-Hydroxy-β-methyl-butyrat vermindert durch Substratinhibition die Verfügbarkeit von Acetyl-CoA, dem zentralen Cofaktor bei der Fettsäure und Cholesterin-Biosynthese. Darüberhinaus hat Coenzym Q in der trocken gelagerten Mischung die Funktion eines die Lagerstabilität fördernden Antioxidans. Im Sinne der vorliegenden Erfindung wird unter 'Q10' die in der Biochemie übliche Unterscheidung der biogenen Ubichinone durch die Länge der Isoprenyl-Seitenketten verstanden, wobei die Spezies mit 10 Kohlenstoffatomen in der Seitenkette die bei den meisten Säugern einschliesslich des Menschen am häufigsten vertretene Spezies ist. Es ist aber auch möglich, die erfindungsgemässe Mischung mit anderen, im Menschen physiologisch wirksamen Ubichinonen herzustellen, wie sie beispielsweise aus Mikroorganismen gewonnen werden können. Beispielsweise überwiegt in Hefe (*S. cerevisiae*) die CoenzymQ-Spezies Q6 (6 Kohlenstoffatome).

Es ist möglich, die erfindungsgemässe Mischung mit weiteren, den synergistischen Effekt vorteilhaft unterstützenden Inhaltsstoffen anzureichern. Eine solche Mischung kann mindestens eine oder, in beliebiger Kombination, mehrere Substanzen aus der Gruppe bestehend aus Vitamin C, Liponsäure, Vitamin E, Ribose, Niacin, Niacinamid, Creatin und Cr(III)Salzen, vorzugsweise Cr-picolinat oder Cr-nicotinat, umfassen. Die Stoffe sind aus der Sport- und Gesundheitsernäherung bekannt und spielen entweder eine Rolle im Energiestoffwechsel bzw. der Fettverwertung, der Mobilisierung der Körperreserven oder haben als zelluläre Antioxidantien Schutzfunktion gegenüber einem gesteigertem Energiestoffwechsel. Insbesondere (-)-Hydroxy-Zitronensäure, die als dominierende Fruchtsäure aus Früchten der Gattung *Garcinia* gewonnen werden kann, ist eine weitere Komponente der Mischung in der am meisten bevorzugten Ausführungsform.

Bevorzugte praktische Ausführungsformen der erfindungsgemässen Mischung, wie auch anderer Salze oder Mischungen der vorliegenden Erfindung, sind an sich bekannte Dareichungsformen wie Kapseln, Dragees, Tabletten, Injektionslösungen, Brausetabletten, die beispielsweise 10-1000 mg Carnitin (oder ein Derivat), 10-1000 mg Coenzym Q und 10-1000 mg β-Hydroxy-β-methyl-butyrat enthalten. Beispielsweise kann eine herkömmliche Hartgelatine-Steckkapsel 300 mg von jeder der drei erfindungsgemässen Substanzen enthalten, vermischt mit Farbstoffen und mikrokristalliner Cellulose als Trägerstoff. Es ist aber auch möglich, diese Substanzen in vergleichbaren Mengen einer geeigneten Portion eines Lebensmittels, beispielsweise Cornflakes, Energy-Bars, Marmelade etc. hinzuzufügen.

Ein weiterer Gegenstand der vorliegenden Erfindung ist Carnitin-Magnesium-Hydroxycitrat. Es handelt sich um ein Salz geringer Hygroskopizität und ist mit dem vorliegenden Verfahren herstellbar, durch Vermischen von Magnesiumhydroxid, Hydroxy-Zitronensäure und Carnitin oder Alkanoyl-Carnitin im Molverhältnis 1:1:1. Es handelt sich um ein Neutralsalz in stöchiometrischer Zusammensetzung, in Hinsicht auf die Ladungsverteilung und die ionisierbaren funktionellen Gruppen. Unter Hydroxycitrat im Sinne der vorliegenden Anmeldung ist vorzugsweise das (-)-Enantiomere zu verstehen, wie beschrieben (Lewis, Y. et al, Water extract of (-)Hydroxy-Citric acid from fruit of *Garcinia Cambogia*, Phytochemistry (1965), 4, p. 619-625; Lewis, Y. et al., Acetone extract of (-)Hydroxy-Citric acid from the fruit of *Garcinia Cambogia*. Das Salz kann vorzugsweise im Sinne des oben gesagten als Schlankheitsmittel, zur Förderung des Abbaus des Körperfettes, benutzt werden. Die Anwendung von Hydroxycitrat als solchem als Schlankheitsmittel und geeignete Dosierungen sind beispielsweise beschrieben in US 5783603. Überraschenderweise unterstützt das neue, erfindungsgemässe Mischsalz mindestens bei oraler Verabreichung in überraschender, mehr als nur additiver Weise das Zustandekommen des erwünschten Fettabbaus bzw. Gewichtsverlusts. Es handelt sich um einen synergistischen Effekt der als Komplexsalz vorliegenden Mischung. Ohne auf die Theorie festgelegt werden zu wollen, ist es möglich, dass dieser Effekt durch eine bessere Bioverfügbarkeit des Komplexsalzes bewirkt wird. HCA neigt zu spontaner Lactonisierung in wässriger Lösung bzw. im Gastrointestinaltrakt. Die Komplexierung mit Mg vor Herstellung einer Lösung könnte bzgl. der unerwünschten Umlagerung zum Lacton stabilisierend wirken und so eine viel höhere Wirksamkeit im Zusammenwirken mit Carnitine bewirken. Zumindestens zeigt das erfindungsgemässe Salz bezüglich der Umlagerung von HCA zum Lacton eine verbesserte Lagerstabilität gegenüber bekannten Salzen von HCA unter im pharmazeutischen Bereich üblichen Standardtestbedignungen. Es ist auch möglich, dass es sich um einen bisher unerkannten stimulierenden Effekt insbesondere des Magnesiums, im Sinne des oben zum Wirkungsmechanismus von Carnitin und Hydroxycitrat Gesagtem, handelt. Allosterisch regulierte Enzyme werden oft von verschiedenen Faktoren, wie z.B. auch Mg-Kationen, reguliert. Magnesium ist zudem für verschiedene Enzyme ein wichtiger Cofaktor bzw. Bestandteil des Holo-Enzyms. Es ist möglich, dass die für Carnitin und Hydroxycitrat wichtigen bzw. dadurch beeinflussten Enzymsysteme einer gleichzeitigen, synergistischen Kontrolle durch Magnesiumionen unterliegen.
Hydroxy-Zitronensäure (HCA) aktiviert indirekt die Carnitin-Palmitoyl-Transferase I (CPT) durch Suppression der Synthese von Malonyl CoA. Malonyl CoA ist ein wichtiger allosterischer Inhibitor für dieses Enzym. CPT ist wichtig für die Transportfunktion von Carnitin für Fettsäuren, über die Mitochondrienmembran hinweg, und kann bei ungenügender Aktivität limitierend für den Carnitin-vermittelten Transport und damit für die Fettsäure-Oxidation in den Mitochondrien sein. Weiterhin ist Malonyl CoA direkter Vorläufer für die Biosynthese von Fettsäuren und Cholesterin.
Der Wirkungsort der HCA ist das Enzyme Citrat-Lyase, das durch HCA kompetitiv inhibiert wird. Die Affinität des Enzyms für HCA ist mehr als 100-fach höher als für das natürliche metabolische Substrat, Zitronensäure. Das Lactonderivat, zu dem HCA spontan cyclisiert, hat demgegenüber eine deutliche geringere Affinität.

Bevorzugte praktische Ausführungsformen der erfindungsgemässen Mischung sind an sich bekannte Dosierungsformen wie Kapseln, Dragees, Tabletten, Injektionslösungen, Brausetabletten, die beispielsweise 10-2000 mg Carnitin-Magnesium-Hydroxycitrat enthalten. Beispielsweise kann eine herkömmliche Hartgelatine-Steckkapsel 1000 mg Carnitin-Magnesium-Hydroxycitrat enthalten, vermischt mit Farbstoffen und mikrokristalliner Cellulose als Trägerstoff. Es ist aber auch möglich, die erfindungsgemässe Substanz in vergleichbaren Mengen einer geeigneten Portion eines Lebensmittels, beispielsweise Cornflakes, Energy-Bars, Marmelade etc. hinzuzufügen. Es ist auch möglich, Carnitin-Magnesium-Hydroxycitrat in Mischung mit β-Hydroxy-β-methyl-butyrat oder anderen der vorher genannten Substanzen zu verwenden.

### Beispiele

### Beispiel 1

### Herstellung von L-Carnitin-Magnesium-Citrat

128 g wasserfreier Zitronensäure und 39 g Mg(OH)₂ pharm. werden in einem Knetgerät für kleine Chargen (HKD-T 0.6 IKA) mit 36 g entionisiertemWasser (Wasseranteil bezogen auf Gesamtmischung: 11.6 %) unter Aufheizen bis 73 °C während 40 min vermischt. 107.5 g L-Carnitin werden dann hinzugegeben und es wird weitere 35 min. bei 40-50 U/min geknetet, bis sich eine gummiartige, weisse Konsistenz ergibt. Nach Trocknen im Vakuum bei 90°C während 4 h ist die Masse trocken und hart. Das Produkt wird mit IR und Polarimetrie geprüft und ist sehr rein. Das Produkt ist in Wasser ohne Rückstand löslich. Der Wassergehalt der getrockneten Substanz ist 10.1 Gew.%.

### Beispiel 2

### Herstellung von L-Carnitin-Magnesium-Citrat

134 g wasserfreier Zitronensäure und 38.5 g Mg(OH)₂ pharm. werden zunächst in einer Fliehkraftmühle während 7 min. vermischt und dann zusammen mit 36 g entionisiertem Wasser (Wasseranteil bezogen auf Gesamtmischung: 11.4 %) in einem Knetgerät für kleine Chargen (HKD-T 0.6 IKA) unter Aufheizen bis 79°C während 30 min. vermischt. 107.5 g L-Carnitin werden dann hinzugegeben und es wird weitere 45 min. bei 40-50 U/min bis zur Homogenität geknetet. Nach Trocknen im Vakuum bei 90°C während 4 h ist die Masse trocken und hart. Das Produkt wird mit IR und Polarimetrie auf Reinheit geprüft und gibt in Wasser eine klare Lösung. Der Wassergehalt der getrockneten Substanz ist 8.8 Gew.%.

### Beispiel 3

### Hygroskopizitätstest

Die mit einem FREEWIT Siebgranulator auf <0.8 mm Komgrösse granulierten Produktmischungen aus Beispiel 1 und 2 werden bei 56% relativer Feuchtigkeit während der angegebenen Zeiten und 25°C unter konstanten Testbedingungen gelagert. Vorgängig wurde die Probe über Phosphorpentoxid zur Gewichtskonstanz getrocknet. Die Feuchtigkeitsaufnahme aus der Luft wird gravimetrisch bestimmt. Die Gewichtszunahme ist in Prozent bezogen auf das Gesamtgewicht der Probe angegeben. Herkömmliches L-Carnitin-Magnesium-Citrat aus dem Produktionsverfahren nach EP 402 755, das zu gleicher Korngrösse pressgranuliert worden ist, dient als Referenzprobe und ist bezüglich der Partikeleigenschaften (Fliessverhalten, Staubarmut) vergleichbar.

| Lagerzeit/h | +Gew.% Referenz | +Gew.% Beispiel 1 | +Gew.% Beispiel 2 |
|---|---|---|---|
| 4 | 5.1 | 2.0 | 2.7 |
| 8 | 9.1 | 2.2 | 3.0 |
| 24 | 18.1 | 4.6 | 5.4 |
| 36 | 17.6 | 4.4 | 5.5 |
| 48 | 20.4 | 5.2 | 6.4 |
| 336 | 15.6 | 5.1 | 6.5 |

### Beispiel 4

### Herstellung von L-Carnitin-Magnesium-(-)-Hydroxycitrat

Die Herstellung erfolgt in dem in Beispiel 2 beschriebenem Verfahren, wobei 83 g Carnitin (freie Base), 137 g (-)Hydroxyzitronensäure HCA-650 77.7%, 30 g Mg-Hydroxid und 55 g Wasser vermischt werden.
Die Gewichtszunahme der erfindungsgemässen, vorgängig getrockneten Mischung nach 24 h betrug unter Standardbedingungen 0.82 Gew.%. Bei 56% relativer Feuchtigkeit betrug die gravimetrisch bestimmte Wasseraufnahme der Mischung ≤ 10 Gew.% nach 30 h, entsprechend ca. 30 Gew.% bezogen auf den Carnitingehalt der Mischung.

## Patentansprüche

1. Verfahren zur Herstellung einer granulierbaren Mischung zur Nahrungsergänzung, wobei die Mischung mindestens eine hygroskopische Substanz umfasst, **dadurch gekennzeichnet, dass**
a) in einer ersten Stufe die feste hygroskopische Substanz mit mindestens einer festen organischen Säure und mindestens einem festem Metallhydroxid ausgewählt aus der Gruppe umfassend Magnesiumhydroxid und Calciumhydroxid gemischt wird, ohne Wasserzugabe oder unter Zugabe von höchstens 15 Gew.% Wasser bezogen auf das Gewicht der Gesamtmischung, und dass
b) in einer zweiten Stufe der Wassergehalt einer resultierenden Mischung durch Trocknung unter 5 Gew.% gesenkt wird, wobei vorzugsweise die resultierende Masse pastös bleibt.

2. Verfahren nach Anspruch 1, **dadurch gekennzeichnet, dass** die hygroskopische Substanz Carnitin, vorzugsweise L-Carnitin, oder ein Carnitinderivat oder deren Salz ist, dass die organische Säure Zitronensäure, (-)-Hydroxyzitronensäure, Vitamin C oder eine andere physiologisch unbedenkliche organische Säure ist.

3. Verfahren nach Anspruch 1, **dadurch gekennzeichnet, dass** die Trocknung eine Vakuumtrocknung ist, und dass die Vakuumtrocknung bei 85-120°C und einem Druck von höchstens 50 mbar erfolgt.

4. Verfahren nach Anspruch 1, **dadurch gekennzeichnet, dass** die in der ersten Stufe erfolgende Vermischung bei einer Temperatur von 50°C-120°C, vorzugsweise bei einer Temperatur von 70°C-120°C, erfolgt.

5. Verfahren nach Anspruch 4, **dadurch gekennzeichnet, dass** der Mischungsanteil des hinzugegebenen Wassers höchstens 3 Gew.%, vorzugsweise höchstens 1 Gew.%, bezogen auf das Gesamtgewicht der Mischung, beträgt und dass die Trocknung eine Vakuumtrocknung ist.

6. Verfahren nach einem der vorhergehenden Ansprüche, **dadurch gekennzeichnet, dass** die resultierende Masse verfestigt ist oder wird und zu Partikeln mit einer Grösse von höchstens 1 mm granuliert wird.

7. Verfahren nach Anspruch 1, **dadurch gekennzeichnet, dass** die organische Säure und das Metallhydroxid in stöchiometrischen Mengen gemischt werden.

8. Verfahren nach Anspruch 2, **dadurch gekennzeichnet, dass** Carnitin, Zitronensäure und Magnesiumhydroxid in der ersten Stufe in stöchiometrischen Mengen gemischt werden und ein metallhaltiges Komplexsalz, nämlich Carnitin-Magnesium-Citrat, bilden.

9. Granulierbare Mischung, umfassend Salze aus Carnitin, vorzugsweise L-Carnitin, mindestens einem Erdalkalimetallkation ausgewählt aus der Gruppe umfassend Magnesium und Calcium sowie einer organischen, physiologisch unbedenklichen Säure, **dadurch gekennzeichnet, dass** die Wasseraufnahme der zuvor getrockneten Mischung bei 56% relativer Feuchtigkeit bei Atmosphärendruck und 25°C nach 24 h bezogen auf das Gesamtgewicht der Mischung höchstens 16 Gew. %, vorzugsweise höchstens 10 Gew.%, beträgt.

10. Mischung gemäss Anspruch 9, **dadurch gekennzeichnet, dass** die Säure Zitronensäure, (-)-Hydroxyzitronensäure oder Ascorbinsäure ist.

11. Mischung gemäss Ansprüchen 9 oder 10, **dadurch gekennzeichnet, dass** die Mischung auch mindestens eine weitere Substanz aus der Gruppe enthaltend Ribose, Niacin oder Niacinamid, beta-Hydroxy-beta-methyl-butyrate, Liponsäure, Coenzym Q 10 und ein Chrom(III) Salz umfasst.

12. Salzverbindung, **dadurch gekennzeichnet, dass** das Salz Carnitin-Magnesium-Hydroxycitrat ist und dass das Magnesium, das Carnitin und das Hydroxycitrat im Molverhältnis 1:1:1 vorliegen.

13. Salzverbindung nach Anspruch 12, **dadurch gekennzeichnet, dass** das Carnitin L-Carnitin ist, und/oder dass das Hydroxycitrat (-)-Hydroxycitrat ist.

14. Salzverbindung nach Anspruch 12, **dadurch gekennzeichnet, dass** die Salzverbindung herstellbar ist in einem Verfahren nach Anspruch 1.

## Claims

1. Method for producing a mixture that can be granulated for dietary supplementation, where the mixture comprises at least one hygroscopic substance, **characterized in that**
a) in a first stage the solid hygroscopic substance is mixed with at least one solid organic acid and at least one solid metal hydroxide selected from the group comprising magnesium hydroxide and calcium hydroxide without addition of water or with addition of not more than 15% by weight of water based on the weight of the complete mixture, and **in that**
b) in a second stage the water content of a resulting mixture is reduced by drying to below 5% by weight, with the resulting composition preferably remaining pasty.

2. Method according to Claim 1, **characterized in that** the hygroscopic substance is carnitine, preferably L-carnitine, or a carnitine derivative or salt thereof, **in that** the organic acid is citric acid, (-)-hydroxycitric acid, vitamin C or another physiologically acceptable organic acid.

3. Method according to Claim 1, **characterized in that** the drying is a vacuum drying, and **in that** the vacuum drying takes place at 85-120°C under a pressure of not more than 50 mbar.

4. Method according to Claim 1, **characterized in that** the mixing which takes place in the first stage takes place at a temperature of 50°C-120°C, preferably at a temperature of 70°C-120°C.

5. Method according to Claim 4, **characterized in that** the content of added water in the mixture is not more than 3% by weight, preferably not more than 1% by weight, based on the total weight of the mixture, and **in that** the drying is a vacuum drying.

6. Method according to any of the preceding claims, **characterized in that** the resulting composition is a solid or is solidified and is granulated to particles having a size not exceeding 1 mm.

7. Method according to Claim 1, **characterized in that** the organic acid and the metal hydroxide are mixed in stoichiometric amounts.

8. Method according to Claim 2, **characterized in that** carnitine, citric acid and magnesium hydroxide are mixed in the first stage in stoichiometric amounts and form a metal-containing complex salt, namely carnitine-magnesium citrate.

9. Mixture that can be granulated, comprising salts of carnitine, preferably L-carnitine, at least one alkaline earth metal cation selected from the group comprising magnesium and calcium and an organic, physiologically acceptable acid, **characterized in that** the water uptake of the previously dried mixture after 24 h at a relative humidity of 56% under atmospheric pressure and at 25°C is not more than 16% by weight, preferably not more than 10% by weight, based on the total weight of the mixture.

10. Mixture according to Claim 9, **characterized in that** the acid is citric acid, (-)-hydroxycitric acid or ascorbic acid.

11. Mixture according to Claims 9 or 10, **characterized in that** the mixture also comprises at least one other substance from the group comprising ribose, niacin or niacinamide, beta-hydroxy-beta-methylbutyrates, lipoic acid, coenzyme Q 10 and a chromium(III) salt.

12. Salt compound, **characterized in that** the salt is carnitine-magnesium hydroxycitrate, and that the magnesium, the carnitine and the hydroxycitrate are present in a molar ratio 1:1:1.

13. Salt compound according to Claim 12, **characterized in that** the carnitine is L-carnitine and/or **in that** the hydroxycitrate is (-)-hydroxycitrate.

14. Salt compound according to Claim 12, **characterized in that** the salt compound can be prepared in a method according to Claim 1.

## Revendications

1. Procédé pour la préparation d'un mélange granulable comme complément alimentaire, le mélange comprenant au moins une substance hygroscopique, **caractérisé en ce que**
a) dans une première étape, la substance solide hygroscopique est mélangée avec au moins un acide organique solide et au moins un hydroxyde de métal solide, choisi dans le groupe comprenant l'hydroxyde de magnésium et l'hydroxyde de calcium, sans addition d'eau ou avec une addition d'au maximum 15% en poids d'eau par rapport au poids du mélange total et
b) dans une deuxième étape, la teneur en eau d'un mélange obtenu est abaissée par séchage au-dessous de 5%, la masse obtenue restant de préférence pâteuse.

2. Procédé selon la revendication 1, **caractérisé en ce que** la substance hygroscopique est la carnitine, de préférence la L-carnitine, ou un dérivé de la carnitine ou son sel, l'acide organique est l'acide citrique, l'acide (-)-hydroxycitrique, la vitamine C ou un autre acide organique physiologiquement inoffensif.

3. Procédé selon la revendication 1, **caractérisé en ce que** le séchage est un séchage sous vide et le séchage sous vide est réalisé à 85-120°C et à une pression d'au maximum 50 mbars.

4. Procédé selon la revendication 1, **caractérisé en ce que** le mélange réalisé dans la première étape est réalisé à une température de 50°C-120°C, de préférence à une température de 70°C-120°C.

5. Procédé selon la revendication 4, **caractérisé en ce que** la proportion de mélange de l'eau ajoutée est d'au maximum 3% en poids, de préférence d'au maximum 1% en poids par rapport au poids total du mélange et le séchage est un séchage sous vide.

6. Procédé selon l'une quelconque des revendications précédentes, **caractérisé en ce que** la masse obtenue est solidifiée ou est granulée en particules présentant une grosseur d'au maximum 1 mm.

7. Procédé selon la revendication 1, **caractérisé en ce que** l'acide organique et l'hydroxyde de métal sont mélangés en des quantités stoechiométriques.

8. Procédé selon la revendication 2, **caractérisé en ce que** la carnitine, l'acide citrique et l'hydroxyde de magnésium sont mélangés dans la première étape en des quantités stoechiométriques et forment un sel complexe contenant le métal, à savoir le citrate de carnitine et de magnésium.

9. Mélange granulable, comprenant des sels de la carnitine, de préférence la L-carnitine, au moins un cation de métal alcalino-terreux choisi dans le groupe comprenant le magnésium et le calcium ainsi qu'un acide organique physiologiquement inoffensif, **caractérisé en ce que** l'absorption d'eau du mélange séché au préalable à une humidité relative de 56% à la pression atmosphérique et à 25°C, après 24 h, est d'au maximum 16% en poids, de préférence d'au maximum 10% en poids, par rapport au poids total du mélange.

10. Mélange selon la revendication 9, **caractérisé en ce que** l'acide est l'acide citrique, l'acide (-)-hydroxycitrique ou l'acide ascorbique.

11. Mélange selon les revendications 9 ou 10, **caractérisé en ce que** le mélange contient également au moins une autre substance choisie dans le groupe constitué par le ribose, la niacine ou le niacinamide, le butyrate de bêta-hydroxy-bêta-méthyle, l'acide lipoïdique, la coenzyme Q 10 et un sel de chrome (III).

12. Composé de sel, **caractérisé en ce que** le sel est l'hydroxycitrate de carnitine et de magnésium et le magnésium, la carnitine et l'hydroxycitrate se trouvent dans un rapport molaire 1:1:1.

13. Composé de sel selon la revendication 12, **caractérisé en ce que** la carnitine est la L-carnitine et/ou l'hydroxycitrate est le (-)-hydroxycitrate.

14. Composé de sel selon la revendication 12, **caractérisé en ce que** le composé de sel peut être préparé par un procédé selon la revendication 1.
